# FASCICULE DE BREVET EUROPEEN

(11) **EP 3 687 632 B1**
(45) Date de publication et mention de la délivrance du brevet: **21.12.2022**
(21) Numéro de dépôt: 18792426.1
(22) Date de dépôt: 25.09.2018
(51) Int. Cl.: A61Q 19/08, A61K 8/9789

(54) **UTILISATION COSMETIQUE DE CELLULES MERISTEMATIQUES DE SYRINGA VULGARIS L. POUR UNE ACTION ANTI-AGE SUR LA PEAU**
KOSMETISCHE VERWENDUNG VON SYRINGA VULGARIS L. MERISTEMATISCHEN ZELLEN ZUM SCHUTZ VOR HAUTALTERUNG
COSMETIC USE OF SYRINGA VULGARIS L. MERISTEMATIC CELLS FOR ANTI-AGEING ACTION ON THE SKIN

(30) Priorité: 27.09.2017 FR 1758964
(43) Date de publication de la demande: 05.08.2020
(73) Titulaire: Laboratoires De Biologie Vegetale Yves Rocher, 56200 La Gacilly (FR)
(72) Inventeur: SENNELIER, Bénédicte, 84450 Saint-Saturnin-Les-Avignon (FR); LAUGIER-CASSIN, Florence, 92500 Rueil-Malmaison (FR); MERVOYER, Catherine, 92100 Boulogne-Billancourt (FR)
(74) Mandataire: Novagraaf Technologies
(86) Numéro de dépôt international: PCT/FR2018/052349
(87) Numéro de publication internationale: WO 2019/063927

(56) Documents cités:
- EP-A1- 2 319 914
- FR-A1- 2 995 533
- DATABASE GNPD [Online] MINTEL; 1 février 2017 (2017-02-01), "Regenerating Anti-Wrinkle Eye Contour Gel", XP002781233, Database accession no. 4600671
- DATABASE GNPD [Online] MINTEL; 1 septembre 2014 (2014-09-01), "Pure Brightening Serum Foundation SPF 20", XP002781234, Database accession no. 2684765

## Description

### Domaine technique

La présente invention se rapporte à l'utilisation cosmétique d'un extrait de cellules méristématiques de *Syringa vu*/*garis L.*

La présente invention trouve ses applications notamment dans le domaine de la cosmétique et de la dermatologie, plus particulièrement de la cosmétique cutanée.

Dans la description ci-dessous, les références entre crochets ([ ]) renvoient à la liste des références présentée à la fin du texte.

### Etat de la technique

La peau est un organe vital à part entière qui se compose de trois tissus distincts, assumant chacun différents rôles grâce à différents types cellulaires et différentes structures.

Le tissu le plus en surface et donc le plus exposé est l'épiderme. Cet épithélium pluristratifié (Malpighien) et kératinisé, se compose de différentes cellules associées à de nombreuses fonctions de barrière et de protection. Les cellules majoritaires sont les kératinocytes qui se divisent dans la couche basale et entament leur différenciation jusqu'à la couche cornée (couche la plus externe) puis sont éliminés par desquamation, en 21 à 28 jours, en moyenne. Le rôle majeur de l'épiderme est d'apporter à la peau, et donc au corps humain, une première ligne de protection contre les agressions extérieures, comme les agressions physiques, chimiques, hydriques et bactériologiques. Cette protection est notamment assurée par les couches les plus différenciées et la couche cornée connue pour ses propriétés hydrophobes, son aspect compacte et étanche. Une bonne différenciation et donc une bonne desquamation sont essentielles pour une surface lisse, homogène et régulière. L'ensemble de ces mécanismes dépend directement des capacités prolifératives des kératinocytes. Or, les stress extérieurs et l'âge ralentissent ce processus qui peut alors devenir plus long et plus irrégulier. La desquamation devenant irrégulière et/ou anarchique, des défauts de lissage et d'homogénéité de surface de la peau s'installent.

En position intermédiaire, le derme est un tissu conjonctif investi majoritairement de fibroblastes et de protéines matricielles donnant à la peau ses qualités de compressibilité et d'élasticité connues. Les modifications de sa texture et de sa composition sont largement responsables des altérations de la peau qui surviennent au cours du vieillissement. Le relief de la couche cornée est, par ailleurs, directement conditionné par la qualité et la densité du derme qui la sous-tend. Plus que l'épiderme, dont le renouvellement est rapide et constant, le derme subit des troubles importants liés à l'âge, par le vieillissement de ses cellules et de sa matière. En vieillissant, les fibroblastes sont de moins en moins réactifs et de moins en moins prolifératifs. Avec l'âge, les synthèses des macromolécules de la matrice ne sont plus assurées par les fibroblastes. De plus, ces macro-molécules constitutives du derme telles que les fibres de collagène, les fibres élastiques, les protéoglycanes et les glycosaminoglycanes (acide hyaluronique notamment) ont tendance à dégénérer et à se fragmenter. Leurs réseaux se désorganisent et se réorientent parallèlement à la jonction dermo-épidermique, notamment sous l'action d'enzymes de dégradations des protéines matricielles, les MMP (Matrix Metallo Proteases), aussi appelées collagénases ou élastases. Ces phénomènes d'altération du tissu dermique provoquent en surface un défaut d'organisation de l'épiderme et, d'une façon plus visible et plus directe, une perte de fermeté pouvant aller jusqu'à la ptose cutanée et/ou la formation de rides.

Au sein de la trame conjonctive, s'intercalent aussi d'autres cellules et structures, tel un important réseau circulatoire et nutritif, constitué des vaisseaux sanguins et des capillaires lymphatiques, ainsi que les annexes épidermiques : cheveux, poils, ongles, glandes pilosébacées et glandes sudoripares, qui prennent naissance dans le derme profond.

L'hypoderme, situé en profondeur et constitué en majeure partie de lobules graisseux (adipocytes), assure une fonction de support primaire, de protection mécanique et thermique et joue aussi un rôle de stockage des réserves énergétiques rapidement mobilisables pour tous les besoins biologiques, comme par exemple le renouvellement cellulaire, la défense de l'organisme ou la contraction musculaire.

Le marché des produits cosmétiques anti-âge est très important et les attentes des consommateurs sont très élevées. Même en terme d'anti-âge, elles s'orientent de plus en plus vers des produits naturels, efficaces et éco-conçus.

Par exemple, la vitamine C est très connue et a fait ses preuves. Comme les autres vitamines, elle est généralement présente dans les produits anti-âge. Même d'origine naturelle, ce composé est très instable et nécessite donc l'ajout de conservateurs ou l'utilisation de dérivés non naturels, peu appréciés des consommateurs.

Le document EP2319914 décrit des préparations de cellules méristématiques comprenant des dérivés d'acide caféique. Parmi les très nombreuses plantes citées, Syringa vulgaris est citée également. Toutefois, aucune donnée expérimentale démontrant un quelconque effet biologique pour un extrait de Syringa vulgaris n'est décrit.

Il reste donc un réel besoin de trouver de nouvelles compositions et/ou composés d'origine naturelle, permettant d'assurer de façon effective un effet anti-âge au niveau des cellules de la peau, de ses annexes et des muqueuses. En particulier, il existe un réel besoin de trouver des composés naturels qui permettent de prévenir, c'est-à-dire inhiber ou, à tout le moins, retarder ou traiter les effets du vieillissement des structures et des cellules de la peau, de ses annexes et des muqueuses, à la fois en stimulant les cellules de la peau et en évitant la dégradation de ses structures.

### Description de l'invention

La présente invention a précisément pour but de répondre à ces besoins et inconvénients de l'art antérieur.

Les inventeurs sont les tout premiers à utiliser un extrait de cellules méristématiques de *Syringa vulgaris L.* permettant précisément de répondre efficacement aux besoins précités. Ils ont en effet découvert qu'un extrait de cellules méristématiques de *Syringa vulgaris L.* possède de manière inattendue un effet anti-âge sur la peau.

De manière surprenante, les inventeurs ont en outre mis en évidence qu'un extrait de cellules méristématiques de *Syringa vulgaris L.* possède de manière inattendue une action protectrice, raffermissante, lissante, anti-ride et/ou anti-relâchement sur la peau.

Les inventeurs ont notamment mis en évidence un effet de l'extrait sur l'expression des matrix metallo-protéases (MMP) par les fibroblastes dermiques, sur le renouvellement des fibroblastes dermiques et/ou sur le renouvellement des kératinocytes épidermiques.

Ainsi, les inventeurs sont les premiers à proposer cet extrait en tant qu'agent cosmétique anti-âge pour améliorer l'aspect de la peau, notamment par la régénération de la peau et/ou le lissage de la peau et/ou l'amélioration de la fermeté, et/ou l'amélioration de l'élasticité et/ou l'amélioration des propriétés biomécaniques de la peau et/ou la lutte contre les effets visibles de l'âge notamment le relâchement et/ou la perte de fermeté et/ou la perte d'élasticité et/ou la formation des rides. Avantageusement, cet extrait permet en outre d'apporter un effet cosmétique sur la surface de la peau, notamment de lissage, et/ou de douceur. L'utilisation de l'extrait selon l'invention permet avantageusement à la peau de présenter un aspect plus jeune.

Ainsi, un premier objet de l'invention se rapporte à l'utilisation cosmétique d'un extrait de cellules méristématiques de *Syringa vulgaris* L. comprenant des cellules méristématiques de *Syringa vulgaris* L. pour une action anti-âge de la peau et/ou de ses annexes et/ou des muqueuses.

Un deuxième objet de l'invention se rapporte à l'utilisation d'une composition cosmétique ou dermatologique anti-âge comprenant un extrait de cellules méristématiques de Syringa vulgaris L. et un véhicule cosmétiquement et/ou dermatologiquement acceptable, ladite composition comprenant 0,00001 à 5 % en poids dudit extrait de cellules méristématiques de Syringa vulgaris L. par rapport au poids total de la composition, pour une action anti-âge de la peau et/ou de ses annexes et/ou des muqueuses.

Un autre objet de l'invention se rapporte à une utilisation d'une composition cosmétique ou dermatologique telle que définie précédemment, dans un produit dermatologique anti-âge destiné à l'amélioration de l'aspect la peau d'un sujet.

*Syringa vulgaris* L. (Oleaceae), également appelé Lilas commun en Français, est un arbuste originaire d'Europe centrale et d'Orient et largement cultivé et introduit en Europe et en Amérique du Nord. Réputé pour son utilisation en ornementation, il est également utilisé en médecine traditionnelle pour sa faculté à traiter les rhumatismes (arthroses) ou encore le diabète (Su et al., Phytochemical and pharmacological progresson the genus Syringa. Chemistry Central Journal, 2015, 9, 2 **([1])**).

L'« extrait », au sens de la présente invention, comprend des cellules méristématiques de *Syringa vu*/*garis* L..

Les cellules méristématiques de *Syringa vulgaris* L. sont obtenues à partir de culture *in vitro* de cellules de *Syringa vulgaris* L. Les technologies de culture cellulaires végétales *in vitro* ont débuté en 1939 avec la démonstration de l'obtention de cals qui sont des amas de cellules indifférenciées appelés également cellules méristématiques. Leur production à l'échelle industrielle remonte aux années 1990 (Trevor A.Thorpe. History of plant tissue culture. Molecular biotechnology, 2007, 37, 2, 169-180 **([2])**). Le principe consiste, à partir d'un échantillon végétal, à dédifférencier les cellules constitutives de l'échantillon, puis induire ensuite la multiplication cellulaire de ces cellules méristématiques. Le but est d'accroître la biomasse afin que les cellules soient récupérées entières, ou fragmentées avec une concentration importante en métabolites d'intérêt.

Les « métabolites », au sens de la présente invention, sont des composés ou molécules produits par une plante ou une partie de plante, notamment des cellules isolées d'une plante. Il peut s'agir de métabolites primaires, par exemples des sucres, protéines/acides aminés et/ou des lipides. Alternativement ou complémentairement, il peut s'agir de métabolites secondaires comme par exemple des polyphénols, des terpènes et/ou d'alcaloïdes (Bruneton J, Pharmacognosie-plantes médicinales, 5eme édition, Lavoisier, 2016 **([3])**).

Les cellules isolées, ou des fragments de cellules, ou des extraits de cellules, peuvent être utilisées en tant qu'actifs cosmétiques. Une étape supplémentaire peut en outre être réalisée dans le but d'extraire et de récupérer les métabolites des cellules sous une forme plus ou moins purifiée. De nombreux avantages existent en ce qui concerne l'utilisation de la culture cellulaire *in-vitro* pour produire des actifs cosmétiques : la possibilité de fragmenter les cellules permet d'avoir avantageusement accès aux macromolécules de la paroi cellulaire et il est également possible de rajouter dans le milieu de culture des précurseurs métaboliques pour induire la production de métabolites et ainsi les concentrer dans le milieu intra-cellulaire (Franck DiCosmo and Masanaru Misawa, Plant cell and tissue culture : alternatives for metabolite production, Biotechnology Advances 1995, 13, 425-453 **([4])**).

Par exemple, les cellules méristématiques de *Syringa vulgaris* L. peuvent être obtenues, mises en culture et conservées selon les méthodes décrites dans les documents EP1736167 **([5])** et EP2319914 **([6])**.

Les cellules méristématiques de *Syringa vulgaris* L peuvent par exemple appartenir à la lignée cellulaire Syringa vulgaris IRB-SV25/B DSMZ, déposée le 15/09/2004 à la DMSZ (Deutsche Sammlung Von Mikroorganismen and Zellkulturen, Inhoffenstr. 7B, D-38124 Braunscheid, Allemagne) sous le numéro DSM 16857 par la société IRB (Instituto di Richerche Biotechnologiche S.p.A, 7 Via Lago di Tovel, 36077 Altavilla Vicentina (VI), Italie). Cette lignée cellulaire est décrite dans le document EP1736167 **([5]).**

L'extrait peut être constitué de cellules entières isolées, ou de fragments de cellules isolées. Avantageusement, l'extrait de cellules méristématiques de Syringa vulgaris L. peut être constitué constitué des parois cellulaires desdites cellules et/ou du contenu cellulaire desdites cellules. De préférence, l'extrait peut être constitué des parois cellulaires desdites cellules et du contenu cellulaire desdites cellules.

Dans la présente invention, le ou les constituants de l'extrait peu(ven)t être issu(s) de la lyse d'une culture cellulaire de cellules méristématiques entières de *Syringa vulgaris* L..

Par exemple, les cellules méristématiques de *Syringa vulgaris* L. peuvent être mises en culture selon la méthode décrite dans le brevet EP 1736167 **([5])** puis lysées de façon à obtenir deux fractions distinctes : une fraction A constituée de fragments de parois cellulaires et une fraction B constituée du contenu cellulaire. Les fractions A et/ou B peuvent éventuellement être mises en suspension dans la glycérine pour obtenir une préparation glycérinée de cellules méristématiques de *Syringa vulgaris* L. Par exemple, la fraction A et la fraction B peuvent être présents dans l'extrait dans un ratio allant de 1 :99 à 99 :1, par exemple être un ratio d'environ 50 :50. Avantageusement, le contenu cellulaire contient tout ou partie des métabolites tels que décrits ci-avant. Un extrait est susceptible d'être préparé selon le procédé comprenant les étapes de :
a) culture cellulaire *in-vitro*, obtention de cellules méristématiques entières,
b) lyse desdites cellules de l'étape a) par procédé haute pression,
c) filtration desdites cellules lysées à l'étape c),
d) association du retentât qui contient les parois cellulaires des cellules lysées et du filtrat qui contient le contenu cellulaire des cellules lysées, et
e) mise en suspension dudit rétentât et dudit filtrat dans la glycérine avec ajout de gomme xanthane et d'acide citrique.

Selon l'invention, les cellules méristématiques de *Syringa vulgaris* L. peuvent être sous toute forme appropriée, notamment dans le cadre d'une utilisation cosmétique ou dans une composition dermatologique. Par exemple, les cellules méristématiques de *Syringa vulgaris* L. peuvent être entières ou lysées. Elles peuvent être mises en suspension sous forme liquide ou être séchées. Sous forme liquide, le solvant utilisé peut être choisi parmi le propylène glycol, le butylène glycol, le méthylpropanediol, le propane-1,3-diol, la glycérine, un solvant eutectique composé de molécules d'origine végétale ou naturelle comme ceux décrit dans le document FR3017292 **([7])** ou un mélange de ces solvants, cette liste n'étant pas limitative. Les mélanges de ces solvants peuvent être réalisés dans toutes les proportions possibles permettant de conserver les effets techniques mentionnés ci-après. Sous forme sèche, la préparation de cellules peut être obtenue par évaporation sous vide, par atomisation, par lyophilisation ou par tout autre moyen approprié connu de l'homme du métier.

On entend par « action anti-âge », au sens de la présente invention, une action de prévention et/ou de retard et/ou de limitation des signes du vieillissement cutané, notamment du vieillissement accéléré (exogène) provoqué par les stress environnementaux, par exemple les ultraviolets (stress photo-induit), ou du vieillissement chronologique (stress endogène). L'action anti-âge peut passer par une action de stimulation des facteurs du cycle cellulaire des fibroblastes, et/ou une action de régénération de la peau et/ou de diminution du relâchement de la peau ou de la perte de fermeté de la peau ou une perte d'élasticité de la peau, notamment une prévention et/ou un retard et/ou une limitation du relâchement, et/ou une action de raffermissement et/ou une amélioration des qualités bio-mécaniques de la peau. Avantageusement, elle peut favoriser un raffermissement des structures de la peau et/ou une diminution de la formation des rides de la peau, de ses annexes ou des muqueuses. Avantageusement, l'action anti-âge est une action de stimulation des facteurs du cycle cellulaire des fibroblastes et/ou des kératinocytes. Par exemple, l'action anti-âge peut être liée à une stimulation de l'expression de l'expression de E2F1 et/ou PCNA par les fibroblastes et/ou les kératinocytes. Avantageusement, la stimulation des facteurs du cycle cellulaire favorise leur renouvellement.

On entend par « améliorer l'aspect de la peau », au sens de la présente invention, tout effet permettant d'obtenir un avantage sur l'aspect de la peau par rapport à l'aspect de la peau avant utilisation de l'extrait selon l'invention.

On entend par « régénération de la peau », au sens de la présente invention, la stimulation métabolique des cellules cutanées épidermiques ou dermiques, dont le renouvellement est directement ou indirectement lié ou cible du vieillissement intrinsèque ou extrinsèque.

On entend par « diminution du relâchement/ de la perte de fermeté/ de la perte d'élasticité » au sens de la présente invention, une action d'inhibition des matrix metallo-protéases (MMP) qui dégradent le collagène et/ou l'élastine de la peau, de ses annexes et/ou des muqueuses. Il peut s'agir d'une action de protection, et/ou de maintien, et/ou de renforcement des fibres existantes dans le derme (collagène et/ou d'élastine) produites par les cellules dermiques. Avantageusement, cette action peut permettre de protéger, de faire perdurer et/ou d'améliorer les qualités biomécaniques du derme, notamment la fermeté, le volume, la densité et la résistance du tissu.

L'utilisation cosmétique de l'invention peut avantageusement permettre d'améliorer les qualités de surface, donc esthétiques, de l'épiderme, comme de lisser la peau et/ou adoucir la peau.

On entend par « lisser la surface de la peau », au sens de la présente invention, la diminution qualitative et quantitative des microreliefs de la peau, notamment de leur profondeur. Il peut s'agir par exemple de la diminution de la profondeur des rides et/ou des ridules.

On entend par « adoucir la peau », au sens de la présente invention, l'obtention d'un effet plus soyeux de la peau au toucher après utilisation de l'extrait, et/ou une diminution de l'effet de rugosité de la peau au toucher.

Alternativement ou en complément, l'action anti-âge peut être une action de restauration, de maintien ou de renforcement de l'hydratation de la peau, de ses annexes ou des muqueuses, cet effet étant distinct et complémentaire des effets précédemment cités.

On entend par « annexes », au sens de la présente invention, les annexes épidermiques qui prennent naissance dans le derme profond, comprenant au moins un élément choisi parmi les poils, les ongles, les glandes pilosébacées et les glandes sudoripares.

On entend par « muqueuse », au sens de la présente invention, notamment les muqueuses externes comme les lèvres.

On entend par « composition cosmétique », dans la présente invention, toute composition à visée cosmétique, c'est à dire esthétique, une composition pouvant être mise en contact avec les parties superficielles du corps humain, par exemple l'épiderme, les systèmes pileux et capillaires. Avantageusement, une composition cosmétique permet, exclusivement ou principalement, de les protéger, parfumer, maintenir en bon état, modifier leur aspect ou en corriger les défauts superficiels.

Dans la présente, on entend par « composition dermatologique » toute composition à visée dermatologique, c'est à dire une composition pouvant être mise en contact avec les parties superficielles du corps humain, pour un traitement de la peau, des muqueuses, et des phanères, comme les ongles, les cheveux, ou les poils. Une telle composition peut comprendre des actifs additionnels autres que l'extrait de cellules méristématiques de *Syringa vulgaris* L., susceptibles d'être qualifiés de dermatologiques ou thérapeutiques, ce qui explique la qualité de « dermatologique » de la composition.

Par « véhicule cosmétiquement ou dermatologiquement acceptable », on entend un véhicule adapté pour une utilisation en contact avec des cellules humaines et animales cutanées, en particulier les cellules de l'épiderme, sans toxicité, irritation, réponse allergique indue et similaire, et proportionné à un rapport avantage/risque raisonnable.

Le véhicule cosmétiquement acceptable peut être choisi parmi l'eau, l'allantoïne, la glycérine, le méthylpropanediol ; cette liste n'étant pas limitative.

La composition peut être obtenue par tout procédé approprié connu de l'homme du métier pour la fabrication d'une composition cosmétique. Il peut s'agir, par exemple d'un simple mélange. Il peut s'agir alternativement, par exemple, d'un procédé comprenant une étape d'incorporation d'une phase interne dans une phase externe au moyen d'un émulseur, par exemple d'une turbine de type rotor-stator. Il peut s'agir également par exemple d'un procédé utilisant la Température d'Inversion de Phase (TIP), ce procédé étant classiquement utilisé par l'homme de l'art pour obtenir des émulsions huile dans eau dont les gouttelettes dispersées sont particulièrement fines, par exemple avec un diamètre de 0,1 à 1 µm.

Selon l'invention, la composition cosmétique ou dermatologique peut comprendre 0,00001 à 5,0 % en poids dudit extrait de cellules méristématiques de *Syringa vulgaris L.* par rapport au poids total de la composition, par exemple de 0,0001 à 5,0 % en poids, ou de 0,001 à 5,0 % en poids, ou de 0,01 à 5,0 % en poids ou de 0,1 à 5,0 % en poids, ou de 1,0 à 5,0 % en poids, ou de 2,0 à 4,0 % en poids dudit extrait de cellules méristématiques de *Syringa vulgaris L.* par rapport au poids total de la composition.

La composition cosmétique ou dermatologique peut se trouver sous toute forme appropriée pour une application cosmétique ou dermatologique. Avantageusement, la composition peut être une composition à usage topique.

Il peut s'agir par exemple d'une composition sous une forme choisie dans le groupe comprenant une émulsion huile dans eau ou eau dans huile ou un mélange de ces émulsions.

Selon l'invention, la composition cosmétique ou dermatologique peut par exemple se trouver sous une forme choisie dans le groupe comprenant un gel aqueux ou hydroalcoolique, une crème aqueuse ou hydroalcoolique et une lotion aqueuse ou hydroalcoolique. Ces formulations utilisables pour la mise en œuvre de la présente invention sont connues dans l'état de la technique par les formulateurs. Dans ces exemples de composition, il suffit d'ajouter l'extrait de cellules méristématiques de *Syringa vulgaris L.* pour obtenir une composition conforme à la présente invention.

Selon l'invention, la composition peut être sous une forme choisie parmi un onguent, une crème, une huile, un lait, une pommade, une poudre, un tampon imbibé, une solution, un gel, un sérum, un baume, un beurre, une lotion, une suspension, un savon ou une émulsion.

L'extrait peut être utilisé dans une composition cosmétique ou dermatologique seul ou en combinaison avec d'autres substances ou ingrédients actifs ou inactifs cosmétiquement ou dermatologiquement. Les substances ou ingrédients inactifs sont ceux qui n'agissent pas cosmétiquement ou dermatologiquement. Il s'agit des éléments de la composition permettant notamment d'accompagner l'extrait, de constituer une formulation particulière, de conserver l'extrait actif dans le temps, cette liste n'étant pas limitative. Il peut s'agir en d'autres termes de tout produit de base pouvant se trouver dans les compositions cosmétiques ou dermatologiques classiques. Par opposition, les substances ou ingrédients actifs sont ceux qui dans l'application cosmétique ou dermatologique visée ont une action esthétique et/ou médicale.

Ainsi, l'extrait de cellules méristématiques de *Syringa vulgaris L.* peut être la seule substance ou ingrédient actif d'une composition, ou il peut être associé à d'autres substances ou ingrédients actifs d'une composition cosmétique ou dermatologique.

Dans le cadre de l'utilisation selon l'invention, l'utilisation s'entend d'une utilisation non-thérapeutique, par exemple pour le traitement des peaux normales, c'est-à-dire des peaux ne présentant pas un état pathologique, à l'exclusion de toute utilisation thérapeutique.

Ainsi, toute utilisation cosmétique selon l'invention sont respectivement des utilisations cosmétiques non-thérapeutiques et procédés cosmétiques non-thérapeutiques.

D'autres avantages pourront encore apparaître à l'homme du métier à la lecture des exemples ci-dessous, donnés à titre illustratif.

### EXEMPLES

### Exemple 1 : Préparation d'extrait de cellules méristématiques de Syringa vulgaris L.

Dans les exemples suivants, la préparation d'une suspension glycérinée de cellules méristématiques de *Syringa vulgaris* L a été préparé comme suit, et conformément au protocole décrit dans le document EP1736167 **([4]).**
1- Culture cellulaire *in vitro* des cellules de la lignée DSM 16857 pour l'obtention de cellules méristématiques entières. Les cellules cultivées sur un milieu solide (GAMBORG B5 dans une agarose à 1% supplémentée avec 20 g / l de saccharose, 2 g / l de peptone végétale, 1 mg / l de kinétine, 1 mg / 1 d'acide naphtalène acétique, 0,2 mg / l d'acide indolacétique à pH 6,5) sont inoculées dans des flacons de 20 x 300 ml, chacun contenant 50 ml de milieu liquide (GAMBORG B5 supplémenté avec 20 g / l de saccharose, 2 g / l de plante peptone, 1 mg / l de kinéine, 1 mg / l d'acide naphtalénacétique, 0,2 mg / l d'acide indolacétique à pH 6,5). Après la fermentation pendant 7 jours, les cultures sont utilisées pour inoculer également des flacons de 1000 ml contenant chacun 250 ml de milieu liquide qui sont ensuite déchargés après une période de 14 jours. La biomasse est combinée.
2- Lyse des cellules par procédé haute pression à l'aide d'un ultraturrax.
3- Filtration des cellules lysées à travers une maille de nylon avec une porosité d'au moins 100 µm.
4- Le retentât et le filtrat sont associés et mis en suspension dans la glycérine (>80% en poids) avec ajout de gomme xanthane (stabilisant) et d'acide citrique (conservateur).

Pour les exemples suivants, les retentât et le filtrat sont associés et mis en suspension dans la glycérine à hauteur de 10%.

### Exemple 2 : Effet d'un extrait de cellules méristématiques de Syringa vulgaris L. obtenus dans l'Exemple 1 sur le renouvellement des fibroblastes dermiques

Les études ont été réalisées sur des fibroblastes dermiques humains normaux, obtenus à partir de plasties abdominales, ensemencés en monocouche dans les conditions de culture adéquates, à savoir dans un milieu DMEM (« Dulbecco's Modified Eagle Médium ») supplémenté à 5% de sérum de veau foetal (SVF), à une température de 37°C, sous atmosphère à 5% de CO₂ et saturée en humidité. Les cellules ont été traitées avec l'extrait de cellules méristématique de Syringa vulgaris L. (à 10-4%, 10-3 % ou 5.10-3%).

Après 4 jours de traitement, la viabilité cellulaire a été évaluée par quantification du formazan soluble issu du clivage du sel de tétrazolium stable (WST1) par des complexes de la mitochondrie.

Les données du tableau I ci-dessous concernent le pourcentage de viabilité des fibroblastes, ayant subi différents traitements.

**Tableau I : Pourcentage de viabilité des fibroblastes**

| Durée | Témoin milieu | Extrait de cellules méristématiques de Syringa vulgaris L. | | |
|---|---|---|---|---|
| Traitement (jours) | base | 10⁻⁴% | 10⁻³% | 5.10⁻³% |
| 4 | 100 | 127 | 125 | 123 |

Cet exemple montre que l'adjonction de l'extrait de cellules méristématiques de Syringa vulgaris L. dans le milieu de culture permet aux fibroblastes de proliférer d'avantage, jusqu'à 27% à 10⁻⁴%. Les fibroblastes étant des cellules peu prolifératives, ce test valide l'intérêt de l'extrait tel que décrit dans l'invention dans le domaine de l'anti-âge.

### Exemple 3 : Effet d'extrait de cellules méristématiques de Syringa vulgaris L. obtenus dans l'Exemple 1 sur l'expression des matrix metalloprotéases par les fibroblastes

Les études ont été réalisées sur des fibroblastes dermiques humains normaux, obtenus à partir de plasties abdominales, ensemencés en monocouche dans les conditions de culture adéquates, à savoir dans un milieu DMEM (« Dulbecco's Modified Eagle Médium ») supplémenté à 5% de sérum de veau foetal (SVF), à une température de 37°C, sous atmosphère à 5% de CO₂ et saturée en humidité. Les cellules ont été traitées avec l'extrait de cellules meristématique de Syringa vulgaris L. pendant 96h à 10-4%, 10-3 % ou 5.10-3%. Puis les surnagents sont récupérés pour être analysés par la méthode "quanti-body array" (Human MMP array 1 Raybio) au regard de la viabilité résiduelle des cellules. Ceci afin de suivre l'influence de l'extrait de cellules méristématiques de Syringa vulgaris L. sur le niveau d'expression des MMP 1, 3, et 10.

**Tableau II : Niveau d'expression des MMP1, 2 et 3 par les fibroblastes**

| | Milieu base | Extrait de cellules méristématiques de Syringa vulgaris L. | | |
|---|---|---|---|---|
| | | 10⁻⁴% | 10⁻³% | 5.10⁻³% |
| MMP1 | 100% | 84% | 81% | 67% |
| MMP3 | 100% | 68% | 80% | 99% |
| MMP10 | 100% | 65% | 88% | 82% |

Le traitement avec l'extrait de cellules méristématiques de Syringa vulgaris L. permet d'inhiber l'expression de la MMP1 jusqu'à 33% à 5.10⁻³%, de la MMP3 jusqu'à 32% à 10⁻⁴% et de la MMP10 jusqu'à 35% à 10⁻⁴%. Ces résultats valident donc l'utilisation de l'extrait dans un soin cosmétique anti-âge en limitant les dégradations de la matrice extracellulaire du derme induites par les MMP.

### Exemple 4 : Effet d'extrait de cellules méristématiques de Syringa vulgaris L. obtenu dans l'Exemple 1 sur l'expression d'ARN messagers codant pour des protéines du cycle cellulaire

Les études ont été réalisées sur des fibroblastes dermiques humains normaux ou sur des kératinocytes épidermiques humains normaux, obtenus à partir de plasties abdominales, ensemencés en monocouche dans les conditions de culture adéquates, à savoir dans un milieu DMEM (« Dulbecco's Modified Eagle Médium ») supplémenté à 1% de sérum de veau foetal (SVF) pour les fibroblastes et dans un milieu kératinocyte-SFM pour les kératinocytes, à une température de 37°C, sous atmosphère à 5% de CO₂ et saturée en humidité. Les cellules ont été traitées avec l'extrait de cellules méristématique de Syringa vulgaris L. pendant 48h à 10⁻³ % ou 5.10⁻³%. Puis les expressions de *E2F1* et *PCNA* ont été évaluées par RT-qPCR sur les ARN totaux extraits des tapis impliquée dans la progression cellulaire de chaque condition expérimentale. Le gène *E2F1* code pour une protéine de la phase G1 (facteur stimulant la transcription de *PCNA* et *CyclinE*). Le gène *PCNA* code pour un cofacteur de l'ADN polymérase et favorise donc la réplication (phase S).

**Tableau III : Niveau d'expression de E2F1 et PCNA par les fibroblastes**

| | Milieu base | Extrait de cellules méristématiques de Syringa vulgaris L. | |
|---|---|---|---|
| Fibroblastes | | 10⁻³% | 5.10⁻³% |
| E2F1 | 100% | 112% | 150% |
| PCNA | 100% | 99% | 146% |

**Tableau IV : Niveau d'expression de E2F1 et PCNA par les kératinocytes**

| | Milieu base | Extrait de cellules méristématiques de Syringa vulgaris L. | |
|---|---|---|---|
| Kératinocytes | | 10⁻³% | 5.10⁻³% |
| E2F1 | 100% | 193% | 163% |
| PCNA | 100% | 131% | 166% |

Le traitement avec l'extrait de cellules méristématiques de Syringa vulgaris L. permet de stimuler l'expression de E2F1 jusqu'à 50% à 5.10⁻³% chez les fibroblastes et jusqu'à 93% à 10⁻³% chez les kératinocytes. Il stimule également l'expression de PCNA jusqu'à 46% à 5.10⁻³% chez les fibroblastes et jusqu'à 66% à 5.10⁻³% chez les kératinocytes. Ces résultats montrent que l'extrait de cellules méristématiques de Syringa vulgaris L. stimule les facteurs du cycle cellulaire à la fois chez les kératinocytes et les fibroblastes favorisant ainsi leur renouvellement.

### Exemple 5 : Formulation de l'extrait de cellules méristématiques de Syringa vulgaris L. dans un soin de type sérum

| **Composants** | **Pourcentage (% en poids)** |
|---|---|
| Eau | qsp 100 |
| Glycérine | 5,000 |
| Jus d'*Aloe barbadensis* | 5,000 |
| Sorbitol | 4,000 |
| Huile de noix de coco | 5,000 |
| Alcool | 2,800 |
| Stéarate de sorbitane | 1,700 |
| Extrait d'*Acer saccharum* | 1,400 |
| Beurre de *Butyrospermum parkii* | 1,100 |
| Extrait d'*Agave tequilana* | 1,000 |
| Alcool stéarylique (Octadécanol) | 0,700 |
| **cellules méristématiques de Syringa vulgaris** | 1,000 |
| Parfum | 0,400 |
| Acide salicylique | 0,300 |
| Gomme xanthane | 0,300 |
| Acide sorbique | 0,150 |
| Soude | 0,120 |
| Acétate de tocophérol | 0,100 |
| Mangiférine | 1 |

### Exemple 6 : Formulation de l'extrait de cellules méristématiques de Syringa vulgaris L. dans un soin de jour

| **Composants** | **Pourcentage (% en poids)** |
|---|---|
| Eau | qsp 100 |
| Glycérine | 5,000 |
| Huile de Coco | 5,000 |
| Huile de *Helianthus annuus* | 3,000 |
| Myristate de myristyle | 2,000 |
| Huile de sésame | 2,000 |
| Acide stéarique | 1,500 |
| Eau d'*Hamamelis virginiana* | 1,100 |
| Stéarate de glycérol AE | 1,000 |
| **cellules méristématiques de Syringa vulgaris** | 0,500 |
| Parfum | 0,500 |
| Eau de *Calendula officinalis* | 2 |
| Gomme xanthane | 0,150 |
| Acide sorbique | 0,100 |
| Allantoïne | 0,100 |
| Acétate de tocophérol | 0,100 |
| Soude | 0,030 |
| Mangiférine | 1,000 |

### Exemple 7 : Formulation de l'extrait de cellules méristématiques de Syringa vulgaris L. dans un soin de nuit.

| **Composants** | **Pourcentage (% en poids)** |
|---|---|
| Eau | qsp 100 |
| Glycérine | 6,000 |
| Huile de soja | 4,000 |
| Huile de noix de coco | 3,000 |
| Stéarate de sorbitane | 1,000 |
| Beurre de *Butyrospermum parkii* | 1,000 |
| **cellules méristématiques de Syringa vulgaris** | 1,000 |
| Parfum | 0,400 |
| Linoléate d'éthyle | 0,300 |
| Gomme xanthane | 0,150 |
| Acide sorbique | 0,150 |
| Allantoïne | 0,100 |
| Acétate de tocophérol | 0,100 |
| Palmitate de rétinol | 0,100 |
| Extrait d'*Hippophae rhamnoides* | 1,000 |
| Soude | 0,060 |
| Mangiférine | 1,000 |

### RÉFÉRENCES BIBLIOGRAPHIQUES

1. Su et al., Phytochemical and pharmacological progress on the genus Syringa. Chemistry Central Journal, 2015, 9, 2.
2. Trevor A.Thorpe. History of plant tissue culture. Molecular biotechnology, 2007, 37, 2, 169-180.
3. Bruneton J, Pharmacognosie-plantes médicinales, 5eme édition, Lavoisier, 2016.
4. Franck DiCosmo and Masanaru Misawa, Plant cell and tissue culture : alternatives for metabolite production, Biotechnology Advances 1995, 13, 425-453.
5. EP1736167.
6. EP2319914.
7. FR3017292.

## Revendications

1. Utilisation cosmétique d'un extrait de cellules méristématiques de Syringa vulgaris L. comprenant des cellules méristématiques de *Syringa vulgaris* L. pour une action anti-âge de la peau et/ou de ses annexes et/ou des muqueuses.

2. Utilisation cosmétique selon la revendication 1, dans laquelle l'action anti-âge est au moins une action choisie parmi la régénération de la peau, la diminution du relâchement de la peau, la réduction de la perte de fermeté de la peau, la diminution de la formation des rides et un lissage de surface de la peau, de ses annexes ou des muqueuses.

3. Utilisation cosmétique selon la revendication 1, dans laquelle l'action anti-âge est une action d'amélioration des qualités de surface de l'épiderme choisie parmi l'action de lisser la peau et/ou adoucir la peau.

4. Utilisation selon l'une quelconque des revendications 1 à 3, dans laquelle ledit extrait est constitué des parois cellulaires desdites cellules et/ou du contenu cellulaire desdites cellules.

5. Utilisation selon la revendication 4, dans laquelle ledit ou lesdits constituant(s) de l'extrait est/sont issu(s) de la lyse d'une culture cellulaire de cellules méristématiques entières de *Syringa vulgaris* L..

6. Utilisation cosmétique selon l'une quelconque des revendications précédentes, dans laquelle l'action anti-âge est une action d'inhibition de l'expression des matrix metalloprotéases.

7. Utilisation cosmétique selon l'une quelconque des revendications précédentes, dans laquelle l'action anti-âge est une action de stimulation du renouvellement des fibroblastes.

8. Utilisation cosmétique selon l'une quelconque des revendications précédentes, dans laquelle l'action anti-âge est une action de stimulation des facteurs du cycle cellulaire des fibroblastes.

9. Utilisation d'une composition cosmétique ou dermatologique anti-âge comprenant un extrait de cellules méristématiques de Syringa vulgaris L. et un véhicule cosmétiquement et/ou dermatologiquement acceptable, ladite composition comprenant 0,00001 à 5 % en poids dudit extrait de cellules méristématiques de Syringa vulgaris L. par rapport au poids total de la composition, pour une action anti-âge de la peau et/ou de ses annexes et/ou des muqueuses.

10. Utilisation d'une composition selon la revendication 9, ladite composition étant sous une forme choisie parmi un onguent, une crème, une huile, un lait, une pommade, une poudre, un tampon imbibé, une solution, un gel, un sérum, un baume, un beurre, une lotion, une suspension, un savon ou une émulsion.

11. Utilisation d'une composition cosmétique ou dermatologique telle que définie dans l'une quelconque des revendications 9 ou 10, dans un produit dermatologique anti-âge destiné à l'amélioration de l'aspect la peau d'un sujet.

## Patentansprüche

1. Kosmetische Verwendung eines Extraktes aus meristematischen Zellen von *Syringa vulgaris* L., die meristematischen Zellen von *Syringa vulgaris* L. enthält, für eine Anti-Aging-Wirkung der Haut und/oder ihrer Anhängsel und/oder der Schleimhäute.

2. Kosmetische Verwendung nach Anspruch 1, wobei die Anti-Aging-Wirkung mindestens eine der folgenden ist: Hautregeneration, Verringerung der Hauterschlaffung, Verringerung des Festigkeitsverlustes der Haut, Verringerung der Faltenbildung und Oberflächenglättung der Haut, ihrer Anhängsel oder Schleimhäute.

3. Kosmetische Verwendung nach Anspruch 1, wobei die Anti-Aging-Wirkung eine Wirkung zur Verbesserung der Oberflächeneigenschaften der Epidermis ist, ausgewählt aus der Wirkung der Glättung der Haut und/oder der Weichmachung der Haut.

4. Verwendung nach einem der Ansprüche 1 bis 3, wobei der Extrakt aus den Zellwänden der Zellen und/oder dem Zellinhalt der Zellen besteht.

5. Verwendung nach Anspruch 4, **dadurch gekennzeichnet, dass** der/den Bestandteil(e) des Extrakts aus der Lyse einer Zellkultur ganzer meristematischer Zellen von *Syringa vulgaris* L. gewonnen wird/werden.

6. Kosmetische Verwendung nach einem der vorhergehenden Ansprüche, bei der die Anti-Aging-Wirkung eine Wirkung der Hemmung der Expression von Matrixmetalloproteasen ist.

7. Kosmetische Verwendung nach einem der vorhergehenden Ansprüche, bei der die Anti-Aging-Wirkung eine Wirkung der Stimulierung der Fibroblastenerneuerung ist.

8. Kosmetische Verwendung nach einem der vorhergehenden Ansprüche, bei der die Anti-Aging-Wirkung eine Wirkung der Stimulierung von Fibroblasten-Zellzyklusfaktoren ist.

9. Verwendung einer kosmetischen oder dermatologischen Anti-Aging-Zusammensetzung, die einen Extrakt aus meristematischen Zellen von *Syringa vulgaris* L. und einen kosmetisch und/oder dermatologisch akzeptablen Träger enthält, wobei die Zusammensetzung 0,00001 bis 5 Gew.-% des Extrakts aus meristematischen Zellen von *Syringa vulgaris* L., bezogen auf das Gesamtgewicht der Zusammensetzung, enthält, für eine Anti-Aging-Wirkung auf die Haut und/oder ihre Anhangsgebilde und/oder die Schleimhäute.

10. Verwendung einer Zusammensetzung nach Anspruch 9, wobei die Zusammensetzung in einer Form vorliegt, die ausgewählt ist aus einer Salbe, einer Creme, einem Öl, einer Milch, einer Salbe, einem Pulver, einem getränkten Kissen, einer Lösung, einem Gel, einem Serum, einem Balsam, einer Butter, einer Lotion, einer Suspension, einer Seife oder einer Emulsion.

11. Verwendung einer kosmetischen oder dermatologischen Zusammensetzung nach einem der Ansprüche 9 oder 10 in einem dermatologischen Anti-Aging-Produkt zur Verbesserung des Aussehens der Haut einer Person.

## Claims

1. Cosmetic use of an extract of meristematic cells of *Syringa vulgaris* L. comprising meristematic cells of *Syringa vulgaris* L. for an anti-ageing action of the skin and/or its appendages and/or the mucous membranes.

2. The cosmetic use according to claim 1, wherein the anti-aging action is at least one selected from skin regeneration, reduction of skin sagging, reduction of skin firmness loss, reduction of wrinkle formation, and a surface smoothing of the skin, its appendages, or mucous membranes.

3. Cosmetic use according to claim 1, wherein the anti-aging action is an action of improving the surface qualities of the epidermis selected from the action of smoothing the skin and/or softening the skin.

4. The use according to any one of claims 1 to 3, wherein said extract consists of the cell walls of said cells and/or the cellular contents of said cells.

5. Use according to claim 4, wherein said constituent(s) of the extract is/are derived from the lysis of a cell culture of whole meristematic cells of *Syringa vulgaris* L..

6. Cosmetic use according to any one of the preceding claims, in which the anti-aging action is an action of inhibiting the expression of matrix metalloproteases.

7. Cosmetic use according to any one of the preceding claims, in which the anti-aging action is an action of stimulating fibroblast renewal.

8. Cosmetic use according to any one of the preceding claims, wherein the anti-aging action is an action of stimulation of fibroblast cell cycle factors.

9. Use of an anti-aging cosmetic or dermatological composition comprising an extract of meristematic cells of *Syringa vulgaris* L. and a cosmetically and/or dermatologically acceptable vehicle, the said composition comprising 0.00001 to 5% by weight of the said extract of meristematic cells of *Syringa vulgaris* L. relative to the total weight of the composition, for an anti-aging action of the skin and/or its appendages and/or the mucous membranes.

10. Use of a composition according to claim 9, said composition being in a form selected from an ointment, a cream, an oil, a milk, an ointment, a powder, a soaked pad, a solution, a gel, a serum, a balm, a butter, a lotion, a suspension, a soap, or an emulsion.

11. Use of a cosmetic or dermatological composition as defined in any one of claims 9 or 10, in an anti-aging dermatological product for improving the appearance of the skin of a subject.
